# EUROPEAN PATENT APPLICATION

(11) **EP 2 905 027 A1**
(43) Date of publication of application: **12.08.2015**
(21) Application number: 13822084.3
(22) Date of filing: 25.07.2013
(51) Int. Cl.: A61K 35/74, A61P 35/00

(54) **USE OF MYCOBACTERIUM BRUMAE TO TREAT BLADDER CANCER**

(30) Priority: 26.07.2012 ES 201231202
(71) Applicant: Universitat Autònoma de Barcelona, 08193 Bellaterra (ES)
(72) Inventor: JULIÁN GÓMEZ, Esther, E-08193 Cerdanyola del Vallès (ES); LUQUÍN FERNÁNDEZ, Marina, E-08193 Cerdanyola del Vallès (ES); SECANELLA FANDOS, Silvia, E-08193 Cerdanyola del Vallès (ES)
(86) International application number: PCT/ES2013/070547
(87) International publication number: WO 2014/016464

(57) **Abstract**

Use of *Mycobacterium brumae* for the treatment of bladder cancer. The present invention relates to the treatment of bladder cancer, preferably superficial, non-invasive bladder cancer by using mycobacteria *Mycobacterium brumae.* Therapy described in this invention is particularly effective in grade 1 (well differentiated) and grade 2 (moderately differentiated) tumor cells. This makes it possible to treat the disease, which is still in an early stage, by reducing the risk of disease progression to a more harmful stage and/or preventing the risk of metastasis.

## Description

### FIELD OF THE INVENTION

The present invention can be encompassed in the field of medicine and especially in the field of oncology. Particularly, the present invention relates to the treatment of bladder cancer, preferably superficial, non-invasive bladder cancer by using *Mycobacterium brumae* (hereinafter *M. brumae*). Therapy described in this invention is particularly effective in grade 1 (well differentiated) and grade 2 (moderately differentiated) tumor cells. This makes it possible to treat the disease, which is still in an early stage, by reducing the risk of disease progression to a more harmful stage and/or preventing the risk of metastasis.

### STATE OF THE ART

*M. brumae* was isolated and described in 1993 by the same research team that has developed the present invention. No infections caused by *M. brumae,* either in humans or animals, have been reported in the literature. *M. brumae* strain, which is defined as biosafety level 1 in all international culture collections (LDG Standards-ATCC), belongs to a group of non-pathogenic environmental mycobacteria. The use of a mycobacterial cell extract from *Mycobacterium phlei* is currently in a phase II study for treatment of non-invasive superficial bladder cancer; however, *M. phlei* cannot be considered as a non-pathogenic mycobacterium since some cases of infection in humans have been described (Khatter et al., 2008; Paul and Devarajan, 1998; Cage and Saubolle, 1997; Aguilar et al., 1989). Furthermore, heat-killed *Mycobacterium vaccae* has been studied as immunostimulator against tuberculosis, asthma and lung cancer (Assersohn et al., 2002; Houdra et al., 1998; Mendes et al., 2002); however, some cases of human infection caused by *M. vaccae* have also been described (Khatter et al., 2008; Hachem et al., 1996).

Bladder cancer is the most common malignancy of the urinary tract. In Europe, the highest incidence occurs in Western Europe, 23.6 in men and 5.4 in women, and in Southern Europe 27.1 in men and 4.1 in women (Babjuk et al., 2008). Throughout the United States in 2008, an estimated 68,810 people were diagnosed with bladder cancer which caused 14,100 deaths (Kresowik et al., 2009). Approximately 75-85% of patients with bladder cancer present with a disease that is confined to the mucosa or submucosa as they are diagnosed. Moreover, after local surgery there is a high level of local recurrence (78%) and progression (45%) (Brandau et al., 2007).

Bladder tumors are classified into different stages depending on the level of tumor invasiveness in the vesicle tissue. This classification is performed following a cystoscopy examination on the patient according to a classification from the International Union Against Cancer (Sobin, et al., 2009):
- Superficial (non-muscle invasive) carcinomas:
   ∘ Stage T0 includes stage Ta (if only epithelium is affected) or TIS (carcinomas in situ)
   ∘ Stage T1 is defined as tumor invading the subepithelial connective tissue (lamina propria) but remains confined to mucosa.
- Infiltrating (invasive) carcinomas. These are differentiated according to the level of involvement of the bladder internal tissue layers:
   ∘ Stage T2, the tumor has invaded the muscle
   ∘ Stage T3, the tumor has invaded the perivesical tissue, or
   ∘ Stage T4, the tumor has invaded peripheral organs, such as prostate, uterus, vagina, pelvic wall or abdominal wall.

In addition to staging system, the World Health Organization (WHO) established in 1973 classification criteria according to the histopathological analysis of tumor. This classification was subsequently revised by WHO/ISUP (International Society of Urological Pathology) (1998) and updated in 2004 (WHO), in which the degrees of cell differentiation (G1, G2 o G3) established in the 1973 classification have been extended and defined. Such classifications are not mutually exclusive, and even the use of both of them is recommended (Alvarez et al., 2007).

Thus we find:
- Tumor G1 (low grade or well-differentiated grade) corresponding to papillomas, inverted papillomas and papillary urothelial neoplasm of low malignant potential (PUNLMP) in the 2004 classification, and low-grade carcinomas.
- Tumor G2 (intermediate grade or moderately differentiated grade) corresponding to noninvasive low-grade papillary carcinomas, and mostly high-grade carcinomas in the 2004 classification
- Tumor G3 (high grade or poorly differentiated grade) having the highest prognostic factors for recurrence and progression, and defined as high-grade carcinoma in the 2004 classification.

Biological therapy with *Mycobacterium bovis* bacillus Calmette-Guérin (BCG) is only performed in the case of superficial tumors, where the risk of recurrence and progression is evaluated according to the results of the pathology examination, and different therapeutic strategies are chosen. (Babjuck et al., 2011):
- In the case of Ta G1 alone, tumor is considered as low risk and thus following transurethral resection (TUR) the patient will only be monitored.
- In the case of Ta G1 multiple, Ta G2, or T1 G1 or G2, superficial intermediate-risk tumor is considered. Local chemotherapy or BCG therapy will be used, or whether these are contraindicated, another immunotherapy will be used. Then the patient will be monitored.
- In the case of T1 G3 with or without tumor *in situ* (TIS), high-risk tumor is considered. BCG therapy will be used, or whether this is contraindicated, another immunotherapy of local chemotherapy will be used. Then the patient will be monitored.

In order to avoid recurrence and progression to more aggressive stages, other strategies are adopted. Within 24 hours after TUR, a dose of intravesical chemotherapy is given (usually mitomycin C) and, in intermediate- and high-risk tumors, live BCG is intravesically administered for 6 weeks, with the possibility of a longer BCG maintenance therapy. BCG administration prevents recurrence and also prevents or, at least, delays tumor progression; however chemotherapy is unable to prevent progression (van Rhijn, 2009; Babjuk, 2008). This protocol established by Morales et al. in 1976 is still the first choice for treating patients with superficial bladder cancer and carcinoma *in situ* (Gontero et al., 2010).

Patients require therefore an effective prophylactic treatment with BCG in order to prevent tumor recurrence and progression and, in addition, follow-up examinations throughout their lives. Consequently, non-invasive bladder cancer is considered a chronic disease that requires frequent monitoring and repeated treatments and has become one of the most costly cancer treatments -from diagnosis to patient's death-(Ploeg et al., 2009).

Thirty years after starting BCG therapy, the mechanisms by which BCG acts as an antitumor agent are not exactly known. A direct activation (inhibition of cell proliferation) is known to take place on tumor cells (Zhang, 1997), as well as an indirect tumor activity involving the immune system stimulation. BCG is firstly phagocytized by antigen-presenting cells resulting in altered gene expression and secretion of cytokines such as IL-6 (pro-inflammatory cytokine) and IL-8 (chemoattractant) (Beavers et al., 2004; Sabin et al., 2008; Sistani et al., 2007), which leads to a massive infiltration. This local inflammatory reaction in the bladder mucosa is characterized by large numbers of T cells (CD4 and CD8) and macrophages, which involves a pro-inflammatory cytokine secretion and often results in a favorable response. The secretion of IL-12 and IFN-a production by BCG-activated monocytes is indispensable for the activation of antitumor cytotoxic cells, such as natural killer (NK) cells, T CD8 cells, and gamma-delta macrophages and lymphocytes (Suttmann et al., 2004).

However, although effective, BCG immunotherapy has its limitations and causes problems that could endanger its use. Toxicity-associated side-effects are present, although they are not generally severe. Most patients showed symptoms of irritability (cystitis), approximately 40% of the patients developed hematuria and 30% fever, malaise and nausea or vomiting (Lamm et al., 2000, Gontero et al., 2010). There were even some cases of BCG-related sepsis (Gonzalez et al., 2003: Nadasy et al., 2008).

The incidence of BCG sepsis was estimated to be 0.4% (Lamm et al., 2000). It should be emphasized that BCG is an attenuated mycobacterium obtained from *Mycobacterium bovis* after 230 serial passages and is given alive. In fact, although BCG therapy may be effective, there is consensus on which not all the patients with non-invasive bladder cancer should be treated because of toxicity risk factors. Tumors with low risk of recurrence and progression are exposed to overtreatment (Babjuk et al., 2008).

BCG is an attenuated mycobacterial strain obtained from 230 serial passages of the pathogenic mycobacterium *Mycobacterium bovis,* which causes disease in animals. Being an attenuated strain BCG is classified as biosafety level 2. That is to say, BCG is a pathogenic agent that can induce disease in humans and animals, but BCG is unlikely to represent a serious hazard to laboratory staff, the community, livestock or the environment. Laboratory exposures may cause serious infection, but effective treatment and preventive measures are available and the risk of spread of infection is limited. Thus, BCG should be handled at least in a level 2 laboratory, where biological safety cabinets are available to minimize the risk from aerosol formation and handling should be performed by qualified personnel.

Thus, due to the absence of alternatives, other than BCG therapy, for treatment of bladder cancer, the aim of the present invention is to find alternative non-pathogenic mycobacteria that prove to be sufficiently effective as to treat bladder cancer without causing unwanted side-effects to patients. The experiments conducted by Yuksel et al., 2011 consist exclusively of adding sonicated mycobacteria to monocytic cell lines and determining the release of cytokines (see "Experimental Procedures"). A list with mycobacteria that induce a significant production of TNF-alpha and/or IL-12 is included in these experiments (Table 2). However, despite the fact that Yuksel et al., 2011, report the induction of cytokine production by the selected mycobacteria, they really do not demonstrate that the production of cytokines is due to bladder cancer therapy. In Yuksel et al., 2011 neither experiment with bladder cell cultures nor with animal models has been carried out demonstrating that the mycobacteria under study are able to treat bladder cancer. In fact, Yuksel et al., 2011 conclude that *"this study is only the first step in developing a new cancer agent. Further study is required including bladder cell cultures and animals models to develop suitable agents"* (page 26). This assertion clarifies the fact that the treatment of bladder cancer referred by Yuksel et al., 2011 is clearly speculative from a theoretical basis. Thus, in the study by Yuksel et al., 2011, treatment of bladder cancer using mycobacteria should not be considered as sufficiently disclosed, because the use of mycobacteria to treat bladder cancer has not been implemented therein. In fact there are several agents capable of stimulating the production of cytokines and, however, they do not exert any antitumor effect on tumor cells. As indicated above, Yuksel et al., 2011 report insufficiently the application of mycobacteria in the treatment of bladder cancer so that a person skilled in the art can reproduce it, since no example of demonstrative experiments is described.

### DESCRIPTION OF THE INVENTION

### Brief Description of the Invention

The present invention solves the above indicated problem of using the mycobacterium *Mycobacterium brumae* (hereinafter *M. brumae*) for the treatment of bladder cancer, preferably superficial (non-invasive) bladder. The therapy described in this invention is particularly effective against cancer cells (grade 1 and 2 tumors). This makes it possible to treat the disease, which is still in an early stage, by reducing the risk of disease progression to a more harmful stage and/or preventing the risk of metastasis.

Therefore *M. brumae* is considered a good candidate to be used in the preparation of a pharmaceutical composition for treatment of bladder cancer.

Thus, the first aspect of the present invention relates to the use of *Mycobacterium brumae* for the preparation of a medicament, preferably the use of *M. brumae* for the preparation of a pharmaceutical composition for treatment of bladder cancer. In a preferred aspect, the present invention is directed to the use of *Mycobacterium brumae* for the preparation of a pharmaceutical composition for treatment of superficial (non-invasive) bladder cancer, particularly treatment of grade 1 and 2 bladder cancer. In an even more preferred aspect, the use of *Mycobacterium brumae* is carried out in combination with at least one cytostatic compound, preferably mitomycin C.

The second aspect of the present invention relates to a pharmaceutical composition comprising the mycobacterium *Mycobacterium brumae* and pharmaceutically acceptable excipients. Among these excipients, suspending agents and stabilizing agents are included, such as water, saline, sucrose, mannose, trehalose, sodium glutamate, glycerol, and non-ionic and ionic polymers such as polyoxyethylenesorbitan monooleate (Tween) or hyaluronic acid, and the like, or a mixture thereof (Jin et al., 2011). In a preferred aspect of the invention, the composition defined in this paragraph comprises *Mycobacterium brumae* in combination with at least one cytostatic compound, preferably mitomycin C.

*Mycobacterium brumae* can be formulated in liquid suspensions or solid forms. Liquid preparations can be solutions for suspension or emulsion in aqueous solutions (water, saline or phosphate-buffered saline (PBS), non-aqueous solutions or both (aqueous suspensions, oil emulsions, water-in-oil emulsions or oil-in-water emulsions, microemulsions, nanoemulsions or liposomes) (Fox et al., 2011; Jin et al., 2011).

Solid carriers include microparticles, nanoparticles, microspheres, minipumps and natural or synthetic biodegradable or non- biodegradable polymers.

The pharmaceutical composition of the present invention can be prepared in formulations, according to knowledge of state-of-the-art pharmaceutical development, in different forms such as: intradermal injection or oral route (capsules, pills or tablets). The medicament can be administered to a mucosa, for example oral, intranasal, gastric, intestinal, vaginal mucosa or urinary tract. Intravesical administration is a preferred form.

The present invention also relates to *Mycobacterium brumae* to be used as a medicament, preferably to be used for treatment of bladder cancer. In a preferred aspect, the type of bladder cancer is superficial (noninvasive), preferably grade 1 and grade 2 tumor.

A final aspect of the present invention relates to a method of treatment for bladder cancer, preferably superficial noninvasive cancer (grade 1 and 2 tumor), comprising administration of a therapeutically effective amount of *M. brumae* or a pharmaceutical composition containing the same to a patient suffering such disease.

### Brief Description of the Figures

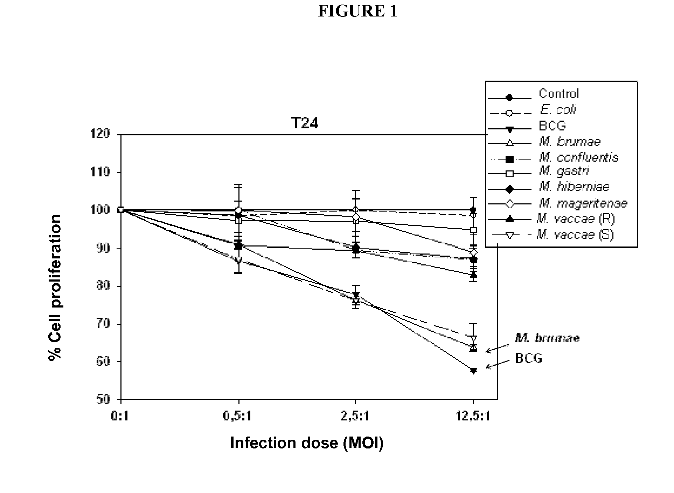

**Figure 1**. Inhibition of dependent proliferation, MOI (multiplicity of infection). Inhibition of T24 cancer line proliferation following infection with different mycobacteria at different MOI. Results are expressed as survival rate versus control (uninfected) cells. Each value represents the mean ± standard deviation of culture triplicates from three independent experiments.

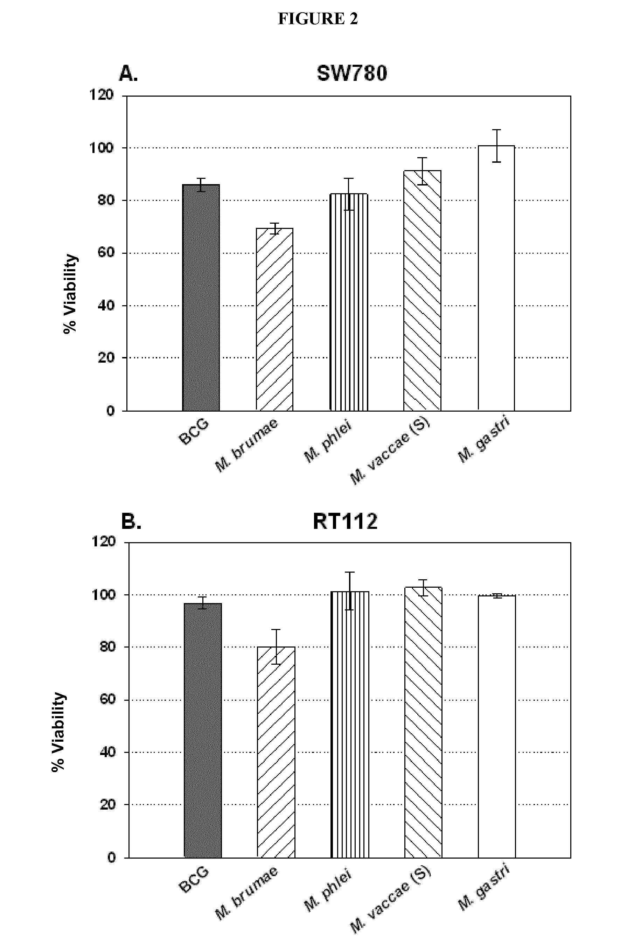

**Figure 2**. Inhibition of proliferation in SW780 (A) and RT112 (B) cell lines. The antitumor effect of *M. brumae* was higher than in the rest of mycobacteria tested, including BCG and *M. phlei.* The infection was induced with live mycobacteria at a MOI of 10:1 for 72 hours. Results are expressed as survival rate versus control (uninfected) cells. Each column represents the mean ± standard deviation of culture triplicates from three independent experiments.

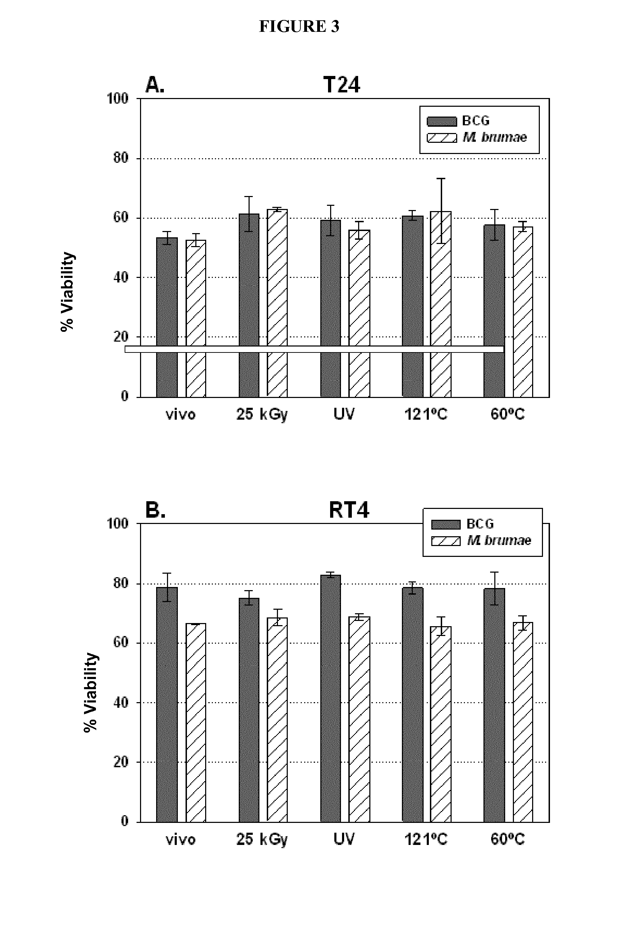

**Figure 3**. Inhibition of tumor proliferation (T24 and RT4 cell lines) with live and dead BCG and *M. brumae* using different treatments. *M. brumae,* even dead, was observed to inhibit tumor proliferation more efficiently than BCG in RT4 cell line. Infection was induced with live mycobacteria, gamma-irradiated (25 kGy) dead mycobacteria, UV-irradiated (20 min) dead mycobacteria and heat-killed (121°C/15 min and 60°C/30 min) mycobacteria at a MOI of 10:1 for 72 hours. Results are expressed as survival rate versus control (uninfected) cells. Each column represents the mean ± standard deviation of culture triplicates from three independent experiments.

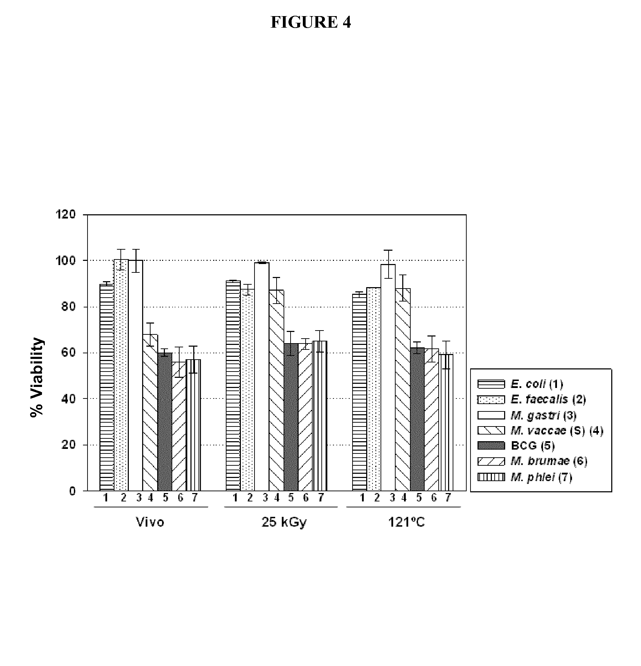

**Figure 4**. Inhibition of tumor proliferation (J82 cell line) with different mycobacteria. BCG, *M. brumae* and *M. phlei* were similarly capable of inhibiting proliferation. Infection was induced with live mycobacteria, gamma-irradiated (25 kGy) dead mycobacteria, UV-irradiated (20 min) dead mycobacteria and heat-killed (121°C/15 min and 60°C/30 min) mycobacteria at a MOI of 10:1 for 120 hours. Results are expressed as survival rate versus control (uninfected) cells. Each column represents the mean ± standard deviation of culture triplicates from three independent experiments.

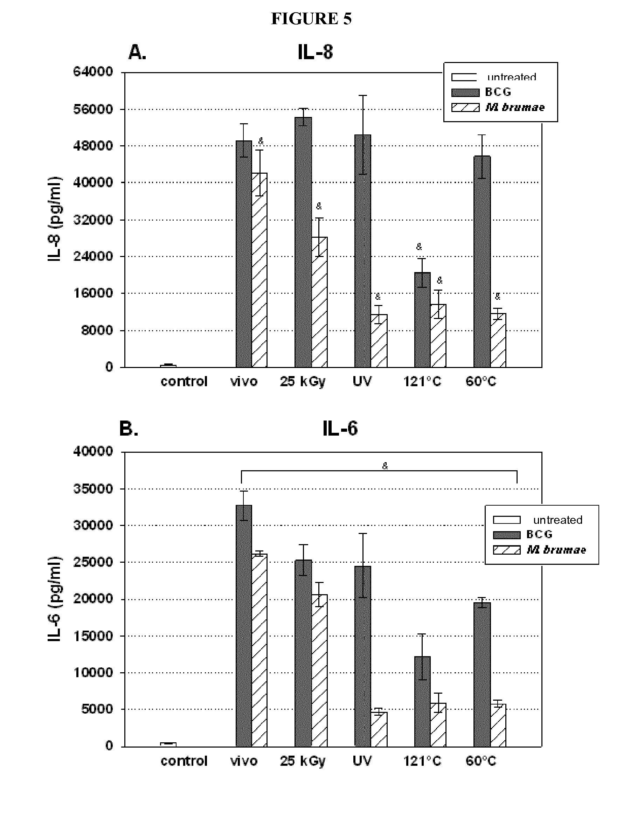

**Figure 5**. Induction of IL8(A) and IL-6 (B) cytokine production in T24 tumor cell line. T24 cell line was infected with both live and dead (killed by irradiation and heat) mycobacteria at a MOI of 10:1 for 72 hours. Results are compared with those of control (uninfected) cells. Each column represents the mean ± standard deviation of culture triplicates from three independent experiments. & p <0.05 vs live BCG.

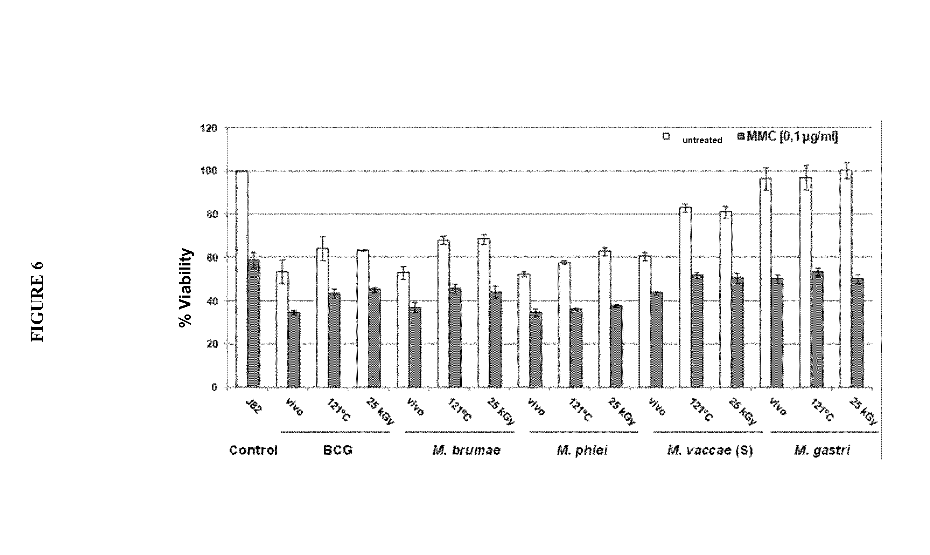

**Figure 6**. Synergistic inhibition effect of tumor growth between mycobacteria and mitomycin C (MMC). The result from J82 cell line is shown. Infection was induced with both live and dead (killed by heat at 121°C/15 min and by irradiation at 25 kGy) mycobacteria at a MOI of 10:1 for 24 hours. The results in *M. brumae* were similar to those observed in BCG. Results are expressed as survival rate versus control (uninfected) cells. Each column represents the mean ± standard deviation of culture triplicates from three independent experiments.

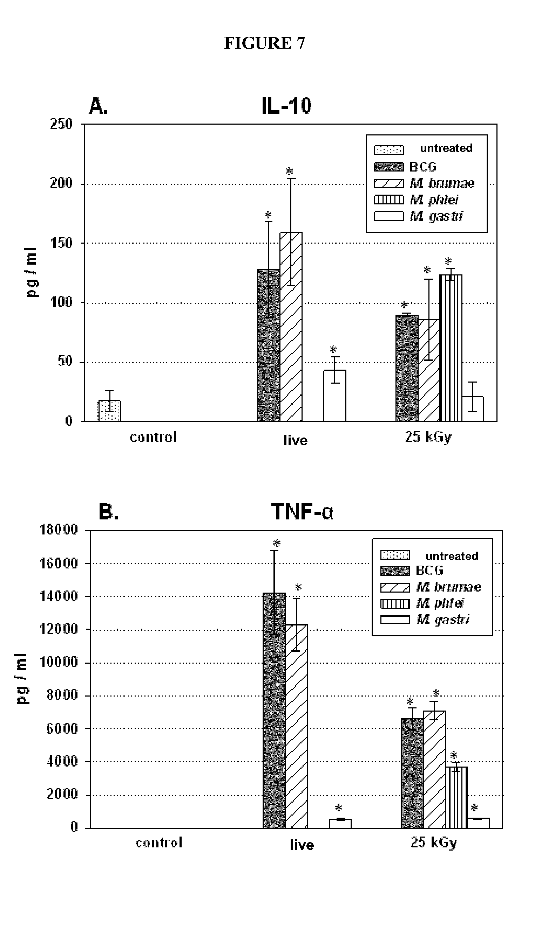

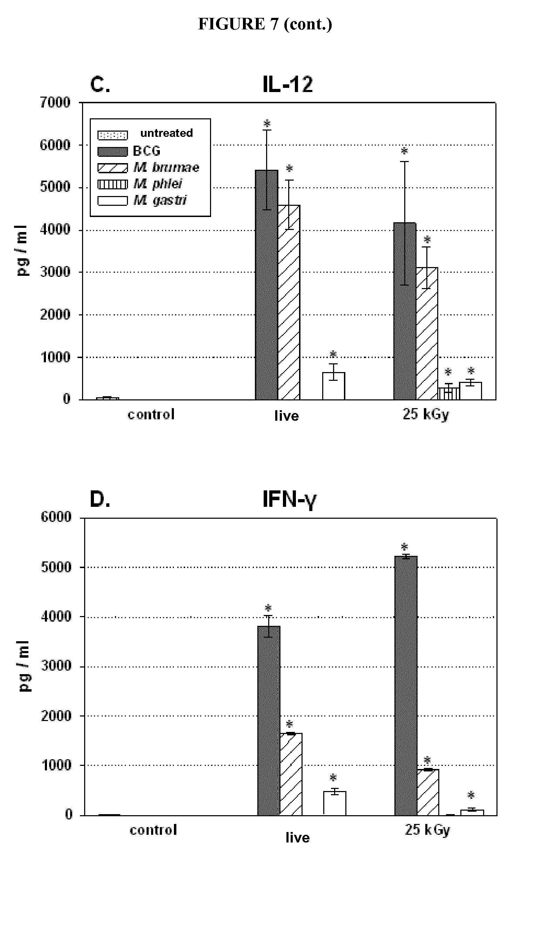

**Figure 7****.** Production of cytokines in PBMCs stimulated with different live and dead (by irradiation) mycobacteria. The production of IL-10 (A), TNF (B), IL-12 (C) and IFN (D) is shown. Results are expressed as the mean ± standard deviation of culture triplicates from three independent experiments. * p <0.05 vs control.

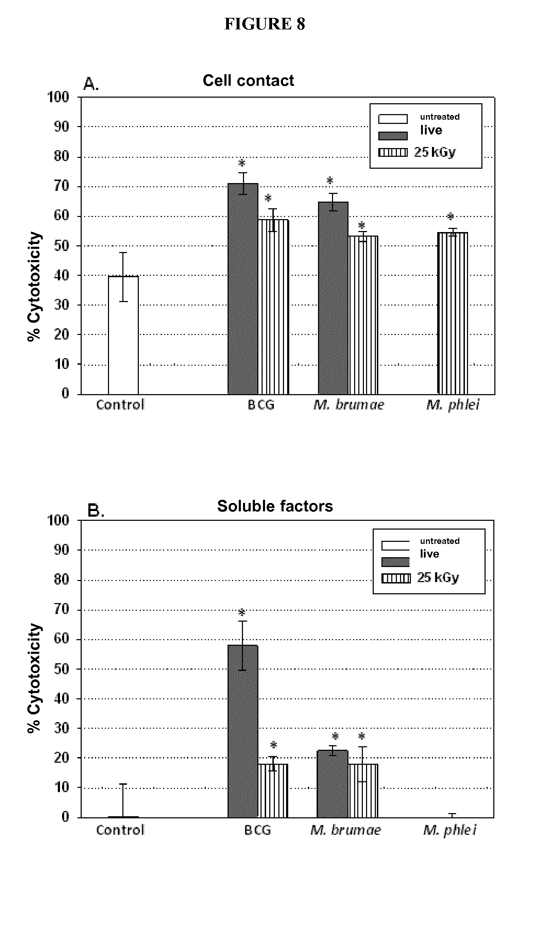

**Figure 8****.** Cytotoxic activity induced by PBMCs stimulated with mycobacteria. The cytotoxic activity of the PBMCs stimulated with mycobacteria is shown in chart A (top) and the cytotoxic activity of soluble factors present in supernatants is shown in chart B (bottom). Results are expressed as the mean ± standard deviation of culture triplicates from three independent experiments. *p<0.05 vs control (uninfected PBMC).

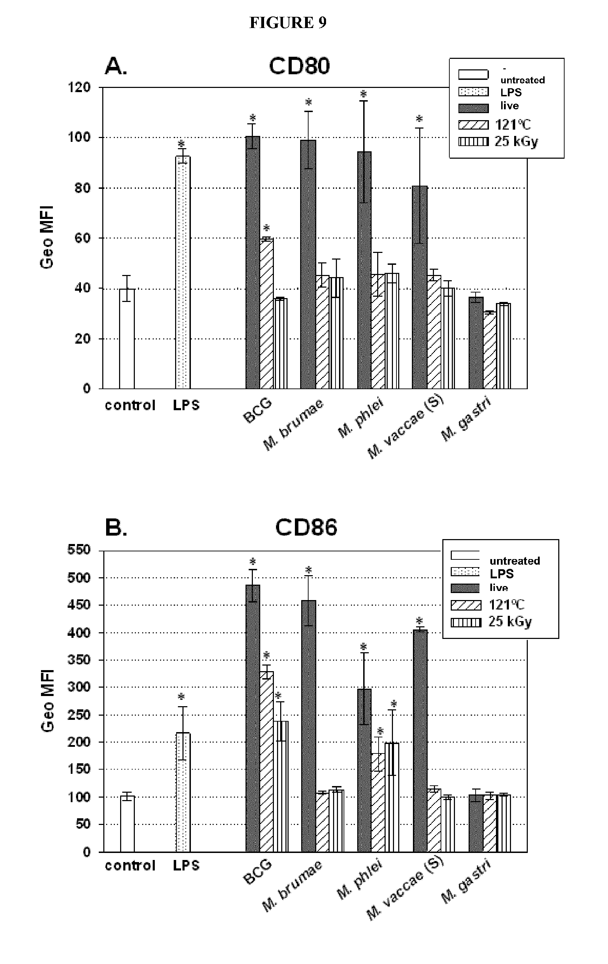

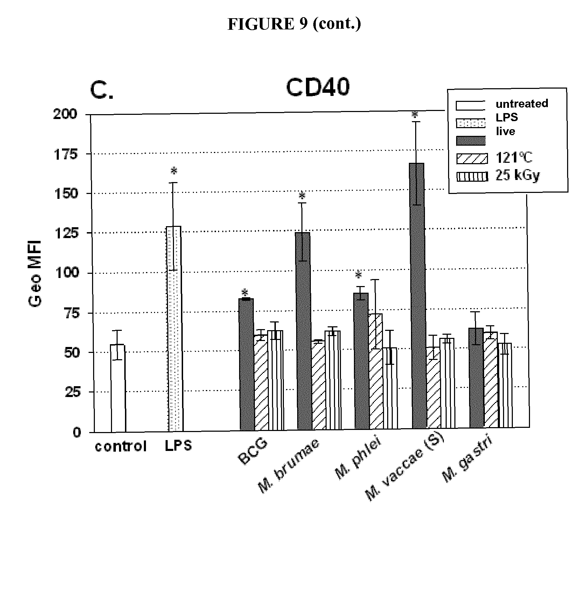

**Figure 9**. Expression of activation markers CD80 (A), CD86 (B) and CD40 (C) in J774 cell line macrophages. Infection was induced with live and dead (heat 121°C and irradiation 25 kGy) mycobacteria. Results are expressed as fluorescence intensity (Geo MFI) mean ± standard deviation of culture triplicates from three independent experiments. *p<.05 vs control (uninfected cells).

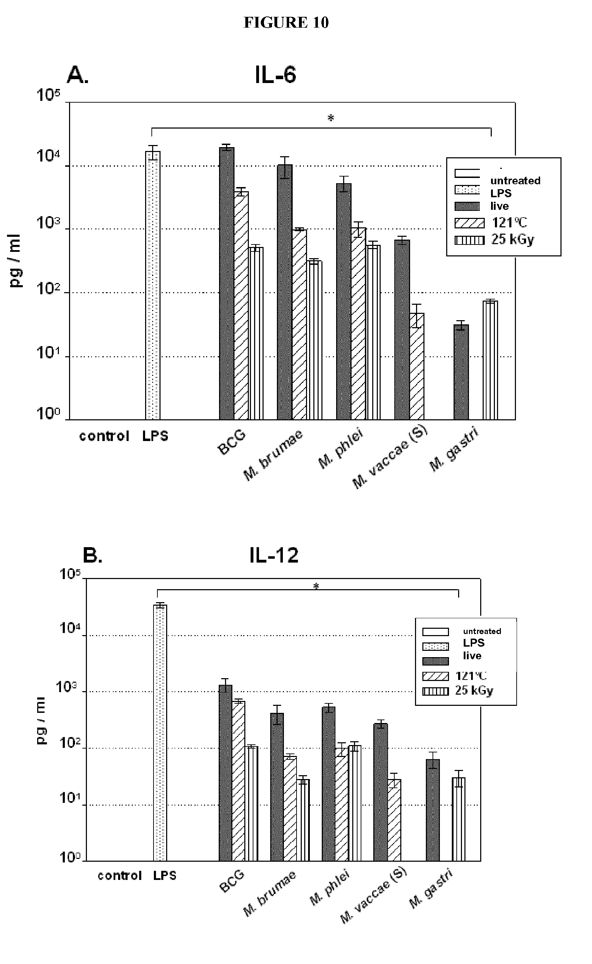

**Figure 10**. Production of cytokines IL-6 (A) and IL-12 (B) in J774 macrophages infected by different live and dead (by heat or irradiation) mycobacteria. Results are expressed as the mean ± standard deviation of culture triplicates from three independent experiments. * p <0.05 vs control (uninfected cells).

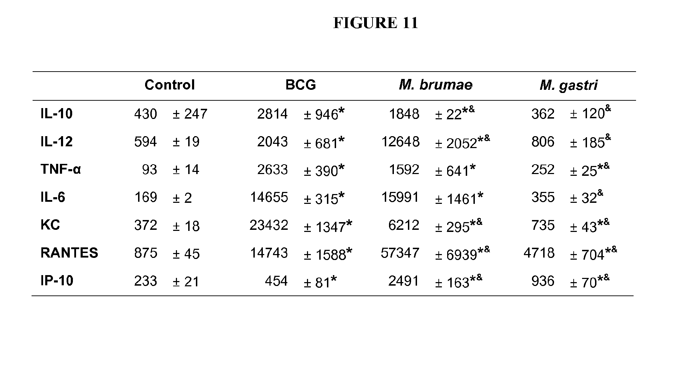

**Figure 11**. Production of cytokines and chemokines in the supernatant of macrophage cultures from mouse bone marrow. Values are expressed in pg/ml. Results are expressed as the mean ± standard deviation of culture triplicates from two independent experiments. * p <0.05 vs control & p <0.05 vs BCG.

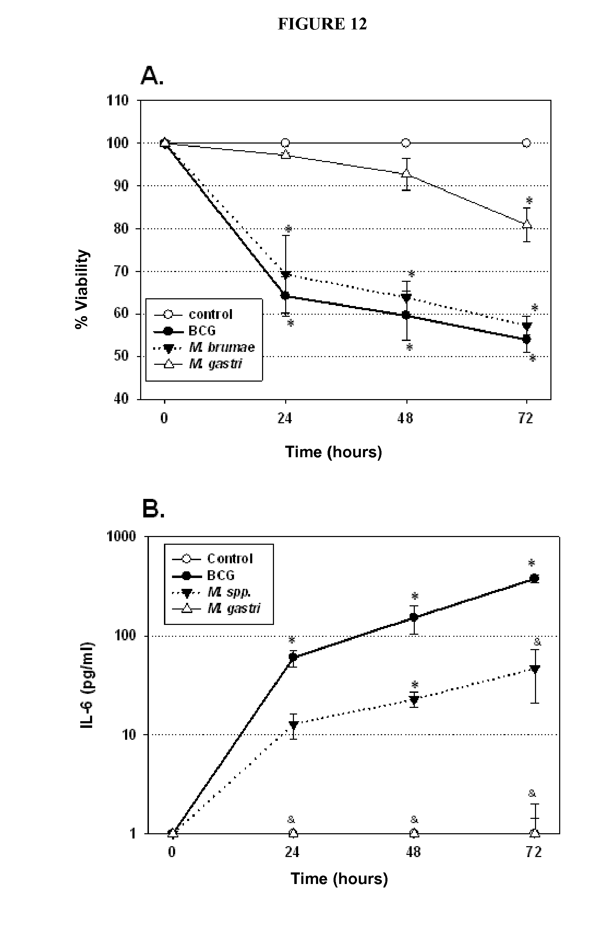

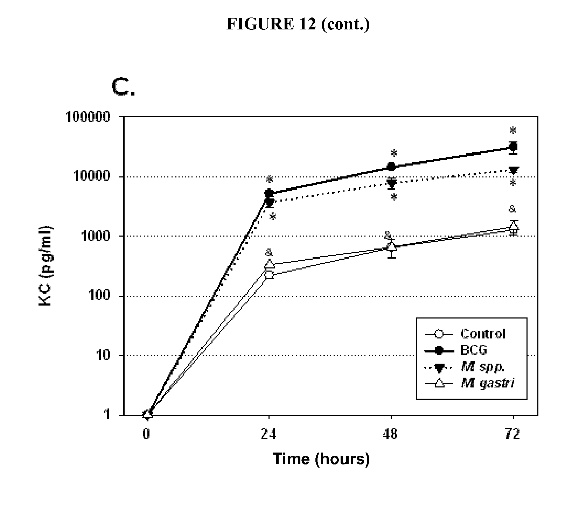

**Figure 12**. Inhibition of tumor proliferation on murine bladder cancer cell line MB49 (A) and induction of IL-6 (B) and KC (similar to human IL-8) (C) production. The inhibition of proliferation was similar between *M. brumae* and BCG being like in human lines time-dependent. *M. brumae* induced elevated IL-6 levels (although lower than BCG) and KC (similar to BCG). Results are expressed as the mean ± standard deviation of culture triplicates from two independent experiments. * p<0.05 vs control & p<0.05 vs BCG.

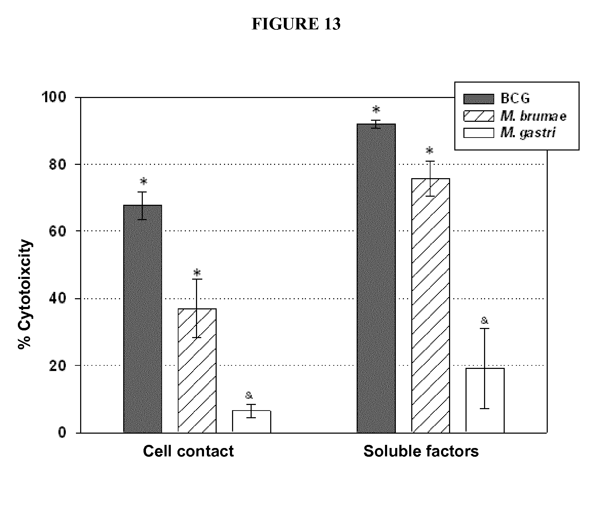

**Figure 13**. Cytotoxic activity induced by bone marrow-derived macrophages stimulated by mycobacteria. The cytotoxic activity of macrophages stimulated by mycobacteria is shown in the left part of the chart and the cytotoxic activity of the soluble factors present in the culture supernatants is shown in the right part of the chart. Similarly, both *M. brumae* and BCG exhibit antitumor activity. Results are expressed as the mean ± standard deviation of culture triplicates from three independent experiments. *p <0.05 vs control (uninfected BMM) & p <0.05 vs BCG.

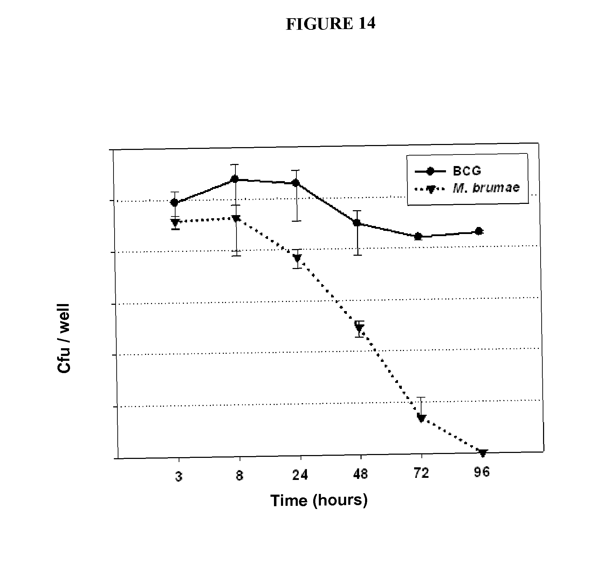

**Figure 14****.** Bacterial survival in murine macrophages (J774 cell line). *Mycobacterium brumae* is removed from the macrophage after 96-hour culture, while BCG survives in the macrophage. Results are expressed as colony-forming units (CFU) on each culture well and correspond to the mean ± standard deviation of three wells. The chart corresponds to the results from one representative experiment of the three independent experiments performed.

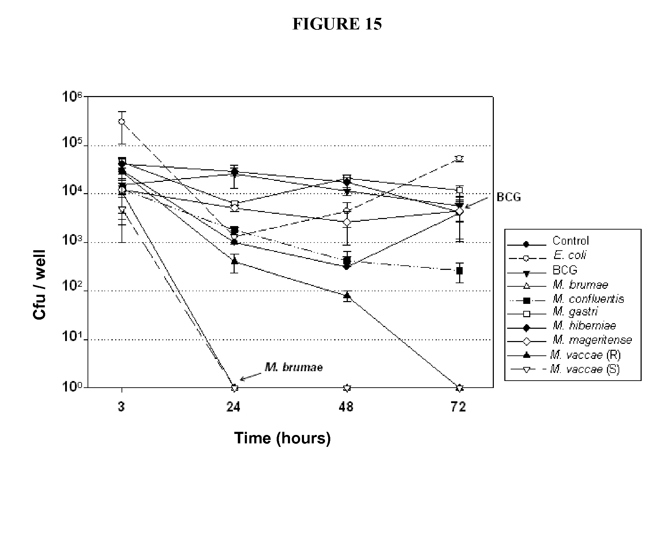

**Figure 15**. Bacterial survival in tumor cell line T24. Unlike BCG and the rest of bacteria under study, *M. brumae* and the smooth colony variant of *M. vaccae* are not viable within the tumor cells at 24 hours post-infection. The rough colony variant of *M. vaccae* decreases its concentration progressively until disappearing after 72-hour culture incubation. Results are expressed as colony-forming units (CFU) on each culture well and correspond to the mean ± standard deviation of three independent experiments.

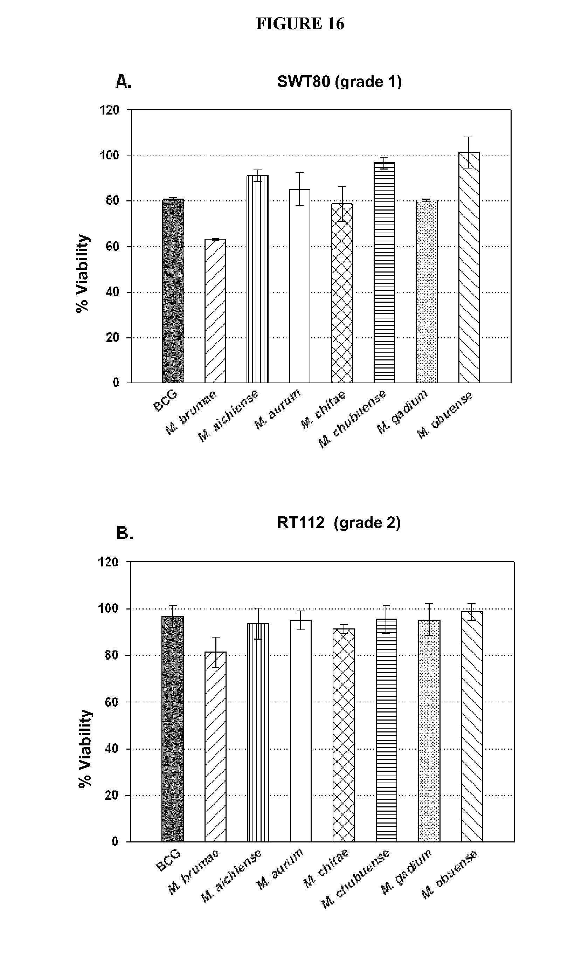

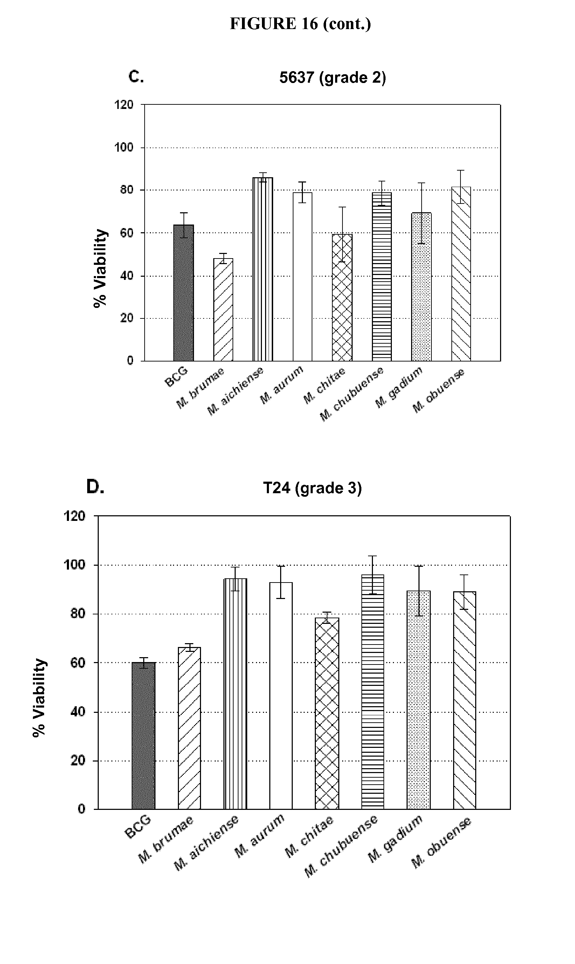

**Figure 16****.** Comparative assays with mycobacteria other than *M. brumae.* The inhibition of T24 (grade 3), RT112 and 5637 (grade 2) and SW780 (grade 1) cell line proliferation is shown following infection with different mycobacteria. Live mycobacteria were infected at a MOI of 10:1 for 72 hours. Results are expressed as survival rate versus control (uninfected) cells. Each column represents the mean ± standard deviation of culture triplicates from at least three independent experiments. Results are discussed in Example 18.

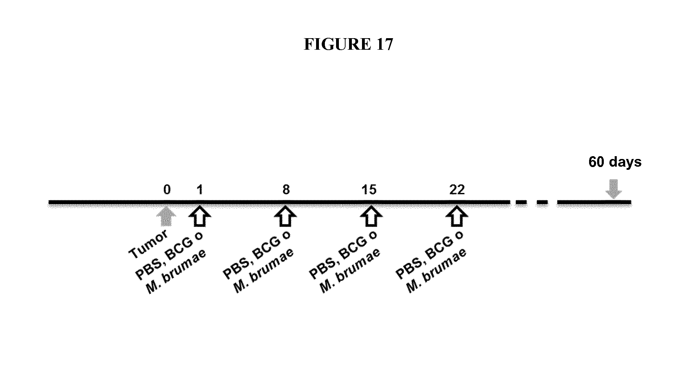

**Figure 17****.** Protocol for tumor induction and animal treatment. Bladder tumor was induced in the animals at day 0. Then, intravesical instillations were performed with suspensions of BCG, *M. brumae* or the carrier used (PBS) on days 1, 8, 15 and 22 after tumor induction.

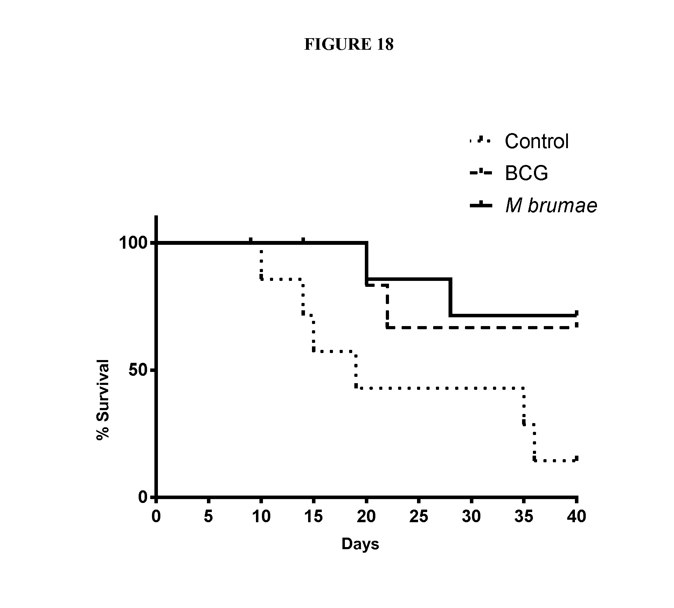

**Figure 18****.** Survival curves (Kaplan-Meier) of mice treated with four intravesical instillations of BCG or *M. brumae* since tumor induction (day 0). The control group received four PBS instillations. As shown in the chart, the animals treated with mycobacteria, both BCG and *M. brumae,* survived to tumor in a percentage significantly higher than that in the animals treated with PBS (p<0.05, long-rank test). Results are discussed in Example 19.

### Detailed Description of the Invention

In the present invention, *M. brumae* (ATCC 51384) was selected from a group of different mycobacteria. *M. brumae* is a non-pathogenic, environmental mycobacterium and its antitumor capacity is similar to that of BCG. In the case of direct antitumor activity, *M. brumae* inhibits grade 3 tumor cells at the same level as BCG or *M. phlei,* but shows a higher antitumor activity than BCG and *M. phlei* in grade 1 and 2 cell lines (an increase in tumor inhibition of 10-20% versus BCG). The direct antitumor activity is maintained when using dead mycobacteria.

As for the indirect antitumor activity on cytokine production in tumor cell lines, *M. brumae,* among all the mycobacteria investigated, is the one which produces higher levels of IL-6 and IL-8 following BCG; in general, the difference is more significant than in the rest of mycobacteria. The production of cytokines decreases when using dead mycobacteria; this fact is also observed for *M. brumae* as well as for the rest of mycobacteria including BCG.

Like BCG, *M. brumae* shows a synergistic antitumor activity with mitomycin C. In the present invention, *M. brumae* proved to stimulate the immune response by inducing the production of pro-inflammatory cytokines by means of three different systems: human peripheral blood, murine macrophage cell line J774 and murine macrophages from bone marrow.

In the case of human cells, cytokine-induced levels (TNF and IL-12) are similar to those induced by BCG, but higher than those induced by *M. phlei* (either TNF or IL-12 or IFN). In the experiments of bone marrow-derived macrophages, *M. brumae* induces a higher production of IL-12, IL-6, RANTES and IP-10 than BCG, and lower of IL-10. It is observed in murine macrophages that *M. brumae* induces the expression of activation markers in the same way as BCG, and even the expression of CD40 even increases as compared to BCG.

Furthermore, in two different experiments where human peripheral blood and bone marrow-derived murine macrophages, *M. brumae* was observed to activate cytotoxic activity against tumor cells in the same manner as BCG. In human cells, this activity induced by the mycobacterium is maintained when using dead mycobacterium.

Moreover, as discussed above, *M. brumae* is not pathogenic. Unlike BCG, *M. brumae* does not survive either in macrophage or tumor cells, and no cases of infection caused by *M. brumae* in humans or animals have been disclosed in the literature.

Finally, it should be emphasized that *M. brumae* is a fast-growing mycobacterium (Luquin, M., et al 1993). Due to the fact that *M. brumae* grows *in vitro* 4 times faster than BCG and in less costly culture media, the large-scale production of *M. brumae* would be more rapid and economical than that of BCG. In addition, the fact that *M. brumae* is a biosafety level 1 mycobacterium makes the preparation process be less complex as compared with BCG.

Therefore, it can be concluded that a non-pathogenic environmental mycobacterium has been identified in the present invention. This mycobacterium is able to inhibit the proliferation of bladder cancer cells and even improves BCG activity. Moreover, as *M. brumae,* unlike BCG, is not pathogenic no unwanted side-effects would be expected to appear.

Furthermore, *M. brumae* promotes a cytotoxic activity on immune system against tumor cells. Thus, *M. brumae,* which is innocuous to man, is an improved alternative in antineoplastic therapy in comparison with BCG.

The Student's t-test was used in the statistical analysis of the results in order to compare the differences from growth inhibition values of the different cell cultures after being infected with bacteria, and the differences from cytokine levels induced by bacteria. The Student's t-test was also used to compare the differences between the expression levels of surface macrophage markers. Differences were considered significant at p < 0.05.

The following examples are illustrative of the invention, and are not to be construed as limiting the invention..

### EXAMPLES

### Example 1. Culture and origin of microorganisms

Eight fast-growing environmental mycobacteria were selected for comparison with BCG which was used as a positive control, and two other bacteria -Gram-positive and Gram-negative- which were used as a negative control.

Such microorganisms were: *Mycobacterium bovis* BCG Connaught (ATCC 35745) acquired by Aventis Pasteur Laboratories (ImmuCyst). *Mycobacterium confluentis* (ATCC 49920) and *Mycobacterium hiberneae* (ATCC 49874) obtained from German Collection of Microorganisms and Cell Cultures. *M. brumae, Mycobacterium gastri* (ATCC 15754), *Mycobacterium mageritense* (ATTC 700351), *Mycobacterium phlei* (ATCC 11758), *Mycobacterium vaccae* (ATCC 15483) (presenting smooth colony morphology), a rough colony morphology variant of *M. vaccae* obtained in our laboratory, *Escherichia coli* (ATCC 10536) and *Enterococcus faecalis* (ATCC 19433) (the last two microorganisms were used as negative controls) obtained from the Collection of Microbial Strains of our laboratory (Laboratory of Mycobacteriology, Autonomous University of Barcelona (UAB), Barcelona, Spain). Mycobacteria were grown in a solid culture medium, i.e., Middlebrook 7H10 agar (Difco Laboratories, Surrey, UK), supplemented with 10% oleic-albumin-dextrose-catalase (OADC) enrichment medium (Sigma-Aldrich, St. Louis, MO, USA) at 37°C. BCG, *M. hiberniae* and *M. gastri* grew for two weeks and *M. brumae, M. confluentis* and *M. mageritensis* for one week. *M. vaccae, E. coli* and *E. faecalis* were grown in solid culture medium of tryptone soya agar (TSA; Scharlau Chemie, Barcelona, Spain) at 37°C for 7, 1 and 1 day, respectively.

### Example 2. Culture of cell lines

Human transitional cell carcinoma cells, T24, J82, RT112, RT4 and SW780, corresponding to histological grades 3, 3, 2, 1 and 1 tumors, respectively, were provided by the Cancer Cell Line Bank of Barcelona Biomedical Research Park (PRBB) (as part of research project: "Thematic Network of Cooperative Cancer Research [RTICC]" funded by the Spanish Ministry of Health, C03/010). Cell monolayers were kept in Dulbecco's Modified Eagle's complete medium (DMEM) / Ham's F-12 nutrient mixture (Gibco, Invitrogen, Carlsbad, CA, USA) supplemented with 10% fetal bovine serum (SBF) (Lonza, Basel, Switzerland), containing 100 U/ml penicillin G (Lab ERN, S.A., Barcelona, Spain) and 100 µg/ml streptomycin (Lab Reig Jofre, S.A., Barcelona, Spain) (complete medium), at 37°C in a 5% CO₂ humidified atmosphere.

The MB49 mouse bladder cancer cell line was kindly donated by Dr. Thomas Tötterman (Rudbeck Laboratory at the Department of Immunology, Genetics and Pathology, Uppsala University, Sweden). Cell cultures of this line were kept in RPMI 1640 complete medium (Invitrogen, Paisley, United Kingdom). The J774 mouse macrophage cell line was kindly donated by Dr. Carlos Martin (Laboratory of Mycobacterial Molecular Genetics, University of Zaragoza, Spain). Cell cultures were kept in DMEM complete medium (Gibco, Invitrogen).

### Example 3. Obtaining of human peripheral blood mononuclear cell (PBMC) culture

Blood samples were obtained from healthy tuberculosis-negative subjects. All donors gave their informed consent for this study.

PBMCs were isolated from heparinized blood samples by Ficoll-Hypaque density gradient technique (Lymphoprep^{™}, Comercial Rafer, Zaragoza, Spain) (Böyum, 1968). Trypan Blue stain was used for cell counting. Then, cells were kept at -80°C until their use or were cultivated in 6-well plates (Nunc), at a concentration of 4 x 106 cells / well in a RPMI 1640 complete medium without antibiotic, at 37°C in a 5% CO₂ humidified atmosphere.

### Example 4. Obtaining of mouse bone marrow-derived macrophages (BMM) culture

C57BL/6 wild type (WT) 10-12 week old strain on C57BL/6 background and TLR2-, TLR4- or MyD88-deficient mice were used. TLR-deficient reproductive mouse couples were obtained from Karolinska Institute (Sweden), with the authorization by Dr. S. Akira (University of Osaka, Japan), and were kept at Arrheius Laboratory Breeding Facility, University of Stockholm (Sweden). All experiments were performed in compliance with the Ethical Guidelines of Stockholm University. The BMMs were obtained as disclosed in the literature (Racoosin et al., 1989; Rothfuchs et al., 2001). In short, after mice were sacrificed, their femurs and tibias from back legs were dissected. The bone marrow cavities were washed in RPMI 1640 medium supplemented with 20% HEPES. The obtained cells were washed and cultured in RPMI complete medium plates to which 20% conditioned medium of L929 cell line (macrophage-colony stimulating factor (M-CSF) source) was added. The cultures were incubated for 7 days at 37°C and in a 5% CO₂atmosphere with medium being replaced every two days. Prior to use, they were washed to remove non-adherent cells and kept in RPMI 1640 culture complete medium for 24 hours..

### Example 5. Preparation of bacterial suspensions

For the infection experiments, bacteria were collected from the culture plate surface and a suspension was prepared in sterile phosphate-buffered saline (PBS) with small crystal spheres to disaggregate bacterial clusters. Then, the tubes were left to stand for 20-30 minutes so that the remaining smaller aggregates could precipitate (Schulze-Röbbecke et al., 1992). Once sedimented, the supernatant was transferred to another tube and then was diluted in PBS until obtaining a concentration equivalent to 1 McFarland turbidity. After centrifugation at 2000 x g for 10 minutes, the bacterial pellet was diluted in complete medium without antibiotics and, then, submitted to three consecutive pulses (45 W) for 30 seconds in a ultrasonic water bath (Bandelin Electronic, Sonorex Super RK52H model, Berlin, Germany) in order to predominantly obtain a cell suspension of individual bacteria (Stokes RW, et al., 2004). Serial dilutions in PBS of representative bacterial suspensions were performed and cultured in solid culture medium plates (Middlebrook 7H10 or TSA). After incubation for 1-4 weeks, depending on the bacterium, colony-forming units (CFU) were determined..

### Example 6. Treatment of bacterial suspensions

Bacterial suspensions in PBS at 1 McFarland turbidity equivalence were submitted to different heating and radiation methods. Heat method consisted in inducing 121°C for 15 minutes in an autoclave, 80°C for 30 minutes and 60°C for 30 minutes in a water bath. Radiation method corresponded to UV light or gamma radiation exposure. Thus, we used the protocol by Shin et al., (2008), and after measuring UV light intensity available in a biosafety cabin of our laboratory, the bacterial suspensions were exposed to UV light for 20 minutes (85 mJ/cm²/s). Exposure to gamma radiation was performed by Aragogamma, S.A., Barcelona, Spain, where bacterial suspensions were submitted to a dose of 5, 15 y 25 KGy according to a method disclosed by Garcia et al., 1987; Gulle et al., 1995.

### Example 7. Infection of cell cultures by bacterial suspensions

For experimental cell line infection, 3 x 10⁴ cells were seeded in triplicate onto 96-well culture plates (Nunc, Roskilde, Denmark) in the case of human cell lines (T24, J82, RT112, RT4 and SW780), and 48-well plates (Costar, Corning, NY, USA) in the case of murine cell line (MB49). After incubation for 3 hours at 37°C in a 5% CO₂ atmosphere, human tumor cells were infected with each bacteria at a multiplicity of infection (MOI) of 0.5:1; 2.5:1; 12.5:1 and 62.5:1 (bacterium : cell), while mouse tumor cells were infected at a MOI of 10:1 after incubation for 24 hours. Cells were incubated at 37°C in a 5% CO₂ humidified atmosphere and at 3 hours post-infection extracellular bacteria were removed by washing three times each well with tepid PBS. Then, complete culture medium (with antibiotic) was added to each well which was incubated at 37°C in a 5% CO₂ humidified atmosphere.

The infection effects by the different bacteria on tumor cells were monitored after 24, 48 and 72 horas for T24, RT112, RT4, SW780 and MB49 cell lines; or after 24, 72 and 120 hours for J82 cell line.

J774 macrophages were placed on 6-well culture plates (Nunc) at a concentration of 5 x 105 macrophage / well. At 24-hour cultivation, they were infected with a MOI of 1:1 for BCG and 10:1 for the rest of mycobacteria. The following steps were the same as those for tumor lines. The infection effects were monitored after 24 hours. The PBMCs were cultivated in 6-well plates (Nunc). 4 x 10⁶ cells / wells were infected with a MOI of 0.1:1 for BCG and 1:1 for the rest of mycobacteria, and then incubated in complete culture medium (without antibiotic) for 7 days at 37°C in a 5% CO₂ humidified atmosphere.

The BMMs were seeded in triplicate onto 48-well culture plates (Costar). At the time of infection, 1 x 10⁵ differentiated macrophages at a MOI of 10:1 were used. The following steps were the same as in the case of tumor lines. The infections effects were monitored after 24 hours.

For BMM cultures, the following compounds were used as positive controls for 24 hours: Pam3Cys-Ser-(Lys)4 trihydrochloride (Pam3) (Enzo Life Sciences, Lausen, Switzerland) at 1 µg/ml, as TLR2 agonist; LPS of *E. coli* (Sigma) at 10 µg/ml, as TLR4 agonist; and Interferon gamma (IFN- gamma) (Mabtech, Nacka Strand, Sweden) at 20 ng/ml, as control of general cell viability since the stimulation type is independent from the TLR agonists.

In all experiments and in parallel to infections, cultures of uninfected tumor cells were used as a control. Each infection was induced in triplicate in each experiment, and the experiments were repeated at least three times.

### Example 8. Reactants for the examination of mycobacteria synergistic effects with a chemotherapeutic agent.

For the examination of mycobacteria synergistic effects with a chemotherapeutic agent, human tumor cells, infected and uninfected, were cultivated together with mitomycin C (Sigma, St. Louis, MO, USA) at a concentration of 10 µg/ml in T24 cell line and 1 µg/ml in J82 and RT4 cell lines for 48 hours.

### Example 9. Cell viability assay

Cell viability was determined by the MTT (3-[4,5-Dimethylthiazol-2-yl]-2,5-diphenyltetrazolium bromide) assay (Mosmann, 1983). At different times after infection -according to the tumor line under study- and as described above, the supernatants were collected and complete culture medium with 10% MTT (Sigma) at a concentration of 5 mg/ml was added to each well and incubated for 3 hours at 37°C in a 5% CO₂ humidified atmosphere. Then, the formed formazan crystals were dissolved with acidic isopropanol and absorbance of each well was determined at 550 nm (Infinite 200 PRO, Tecan, Männerdorf, Switzerland).

### Example 10. Cytokine analysis

Culture supernatants, which were collected at different times after infection according to the tumor line under study and as described above, were centrifuged at 1000 x g for 10 minutes and kept at -40°C until use. The different cytokine levels present in supernatants were determined by a commercial enzyme immune-assay in compliance with the manufacturer's instructions. In human tumor cell lines, interleukin-6 (IL-6) and interleukin-8 (IL-8) tests (Becton Dickinson (BD, Pharmingen, San Diego, CA, USA) and Tumor Necrosis Factor-alpha (TNF-alpha) (Mabtech) assay were used. In PBMCc, IL-6, IL-8 and Interferon gamma (IFN-gamma) (BD), TNF-alpha, IL10 and IL-12 (total) (Mabtech) tests were used. In the mouse tumor line, TNF-alpha, IL-6 and keratinocyte Chemoattractant (KC) (R&D Systems, Minneapolis, MN, USA) tests were used. In J774 cell line, IL10 and IL-12 (total) (Mabtech), IL-6 and TNF-alpha (BD) test were used. In BMMs, KC, Regulated upon Activation, Normal T-cell Expressed, and Secreted (RANTES), Interferon gamma-induced Protein 10 (IP-10) and TNF-alpha (R&D Systems), IL-10 and IL-12 (total) (Mabtech) tests were used. The absorbance results obtained were transformed into concentration of each cytokine after being compared to the standard curve provided by the manufacturer -included in each plate (Costar). Data were expressed as average for duplicates of each triplicate well of experiments.

### Example 11. Viability of mycobacteria in infected cultures

With the aim of determining the intracellular viability of mycobacteria after infection, cultures with T24 cell line were performed, which were sacrificed at different post-infection times (3, 24, 48 and 72 hours). After collecting the culture medium from wells, 400 µl/well of 0.1% Triton X-100 (Sigma) was added in PBS and incubated for 15 minutes at 37°C. Serial dilutions were performed, seeded onto culture plates for bacteria and incubated at the corresponding temperature for 1-2 weeks. Following this time period, count of colony-forming units was performed (CFU) (Dobos et al., 2000).

### Example 12. Detection of surface markers on activated J774 macrophages

For examination of activation markers, surface J774 cells were recovered from wells and incubated with anti-mouse CD32/CD16 antibody (receptor FcγII/III). After 15 minutes the corresponding anti-mouse antibody, diluted in PBS was added. IA^{d} (MHC II), CD40, CD80 (B7.1), CD86 (B7.2) antibodies and their respective isotype controls were used. All antibodies were obtained from Becton-Dickinson. Incubation was performed in ice for 30 minutes. Finally, they were washed and resuspended in PBS to be analyzed by flow cytometry (FACSCalibur) using CellQuestPro software (Becton Dickinson, Heidelberg, Germany). J774 macrophages were stimulated with Lipopolysaccharide (LPS) of *E. coli* (Sigma) at 10 µg/ml for 24 hours and it was used as a positive control.

### Example 13. Cytotoxicity of activated PBMCs and BMMs

The PBMCs were infected with different mycobacteria at a MOI of 0.1:1 for BCG and 1:1 for the rest of bacteria, and the BMMs at a MOI of 1:1, for 7 days and 24 hours, respectively. Then, the activated PBMCs were cultivated with T24 tumor cell line and the activated BMMs with MB49 tumor cell line, at a proportion of 20:1 (effector cell: tumor cell) for 48 and 24 hours, respectively.

In order to analyze the cytotoxic capacity of soluble factors from the culture medium of activated effector cells, supernatant was collected at 24 hours post-infection, centrifuged at 1000 x g for 10 minutes, and used a culture medium for the corresponding tumor cell lines for 48 hours.

After the necessary culture times, tumor cells were collected from each well and cellular viability was determined by MTT assay in the case of T24 cells and by microscopic cell count (Tripan blue stain) in the case of MB49 cells.

### Example 14. Selection of mycobacteria

After selecting a group of 8 mycobacteria among more than 120 species disclosed so far, their direct antitumor capacity was studied on bladder tumor cell lines, as well as their ability to activate an immune response, which is a key factor for indirect antitumor activity.

*M. brumae* was among the mycobacteria studied. The use of a cell extract from another mycobacterium, *Mycobacterium phlei,* for treatment of superficial non-invasive bladder cancer is currently in a Phase II study. That is why *M. phlei* was included in the study although this mycobacterium could not be considered as non-pathogenic, since infection in some human cases had been described. Following a first selection, in most of subsequent experiments *M. vaccae* and *M. gastri* were studied as control in parallel with *M. brumae,* BCG and *M. phlei.* As *M. gastri* was one of the mycobacteria that did not show any antitumor effect in the first experiments, it was selected as a negative control for the rest of experiments.

### Example 15. Direct antitumor response

Only some non-pathogenic mycobacteria are able to inhibit cell growth in bladder cancer. We studied cultures from grade 1 (RT4 and SW780), grade 2 (RT112) and grade 3 (T24 and J82) tumor cell lines, which are relevant to superficial bladder cancer. After being the different mycobacteria infected, it was observed that not all of them produced the same effect. While most mycobacteria did not exert any type of activity on tumor cell, one of them, *M. brumae,* inhibited tumor cell proliferation in the same way as BCG on tumor cell lines. Thus, inhibition values were 30-40%, which even surpassed the antitumor capacity of BCG in grade 1 and grade 2 (RT4, SW780 and RT112) cell lines (Figures 2 and 3). Inhibition of cell proliferation was determined at different times and at different MOI, concluding that the antiproliferative activity was time-dependent and MOI-dependent..

Dead *M. brumae* maintained its capacity for inhibition of tumor cell proliferation. As described above, the use of live BCG in patients can lead to a possible infection by this microorganism. In view of the advantage that might involve the therapeutic use of non-viable bacilli, the direct antitumor capacity of dead mycobacteria was studied through different types of heat and radiation methods. Behavior was similar to that obtained with live mycobacteria. *M. brumae,* even dead, continues to inhibit tumor cell proliferation (Figures 3 and 4).

In addition, *M. brumae* induced the production of cytokines in tumor cell lines. The induction of cytokine production by mycobacteria in the infected tumor cell lines was examined. A direct relationship was observed between the increased production of cytokines and the incubation time, and between the production of cytokines and the concentration of bacteria used for culture infection. The production of cytokines differed depending on the bacteria used and the cell lines studied.

Among live mycobacteria, *M. brumae* induced lower IL-6 and IL-8 values than BCG, similar T24 values to BCG (Figure 5), but higher than the rest of mycobacteria studied.

Furthermore, it should be emphasized that a synergistic effect was produced in the inhibition of mycobacterial tumor proliferation with mitomycin C. Tumor cells were infected with the different mycobacteria and treated in parallel with the chemotherapeutic agent, mitomycin C, which is often used in the treatment of superficial (non-invasive) bladder cancer patients. A synergistic effect was observed between the mycobacterium and the chemotherapeutic agent in the inhibition of tumor cell proliferation. This synergistic effect could be observed when using both live and dead mycobacteria (Figure 6).

### Example 16. Direct antitumor response

The stimulation of human immune system and cytotoxic activity against tumor cells were studied. Thus, human peripheral blood mononuclear cells (PMBC) were infected with both live and dead (irradiated) *M. brumae,* BCG and *M. gastri* (as negative control), as well as with dead (irradiated) *M. phlei* in order to examine their immune-stimulating effect. The presence of pro-inflammatory cytokines (TNF, IFN and IL-12) and antiinflammatory cytokines (IL-10) was evaluated in culture supernatants (Figure 7).

Both live and dead (irradiated) *M. brumae* induced similar IL-10, TNF and IL-12 levels as BCG; only IFN levels were lower than BCG. In all the cases the production of cytokines was higher than that induced by *M. gastri* (Figure 7).

By comparing the infection caused by dead (irradiated) *M. phlei, M. brumae* induced higher TNF, IL-12 and IFN levels, i.e. the three pro-inflammatory cytokines studied. In human cells, *M. phlei* induced no production of either IL-12 or IFN, which is a key cytokine for activation of antitumor response (Figure 7).

The cytotoxic activity of PBMCs -stimulated with different mycobacteria- against T24 tumor cell lines was examined. Both the cytotoxic activity of cells (PBMCs were contacted with T24 cell line) and the cytotoxic activity of soluble factors present in the supernatant of stimulated PBMCs (the supernatant was contacted with T24 cell line) were determined. The PBMCs stimulated with *M. brumae* (both live and dead by irradiation) were observed to exert cytotoxic activity against T24 human tumor cell line, being cytotoxicity values similar to those obtained with PBMCs stimulated with BCG or *M. phlei* (in the case of *M. phlei* only killed by irradiation, live *M. phlei* could not be used) (Figure 8).

Moreover, *M. brumae* was shown to induce soluble factors, which are present in the supernatant of stimulated PBMC cultures, with cytotoxic activity. Cytotoxicity values were lower than those obtained by live BCG, but similar to those obtained by dead (irradiated) BCG (Figure 8). *M. phlei* did not induce any soluble factors in the cultures with cytotoxic activity.

Furthermore, an assay for murine macrophage activation was performed by means of the expression of activation markers and production of cytokines. Thus, several experiments were carried out with J774 murine macrophage cell line (it was not possible to perform these experiments with human blood cells) in order to study in detail the capacity of immune system activation.

Thus, the capability of activation of antigen-presenting cells was studied. It was observed that the expression level of certain superficial markers associated to activation (CD80, CD86 y CD40) increased after infecting the macrophages with the studied mycobacteria (Figure 9). With the exception of CD86 expression, which was observed to have been relatively increased by using dead mycobacteria, it was necessary to infect the macrophages with live mycobacteria in order to find a significant increase in the expression of activation markers. The expression of CD40 was higher than BCG, and the expression of CD80 and CD86 was similar to BCG *after M. brumae* induction (Figure 9).

In the cultures of J774 murine cell line, the production of cytokines after being infected with mycobacteria could also be studied. Thus, *M. brumae* was observed to induce the production of pro-inflammatory cytokines (IL-12 and IL-6) in the infected macrophages. The production of cytokine levels induced by live *M. brumae* were similar (IL-6) to or lower (IL-12) than those induced by BCG (Figure 10). When the macrophages were infected with dead mycobacteria, the production of cytokine levels was lower in all the cases (Figure 10). It should be emphasized that *M. brumae,* in the same way as BCG, induced the production of IL-12, a key factor in bladder cancer immunotherapy, which had already been observed in human peripheral blood cultures (Zaharoff, 2009).

Likewise, the activation of mouse bone marrow macrophages was studied by induction of cytokine/chemokine production and cytotoxic activity against tumor cells.

Thus, macrophages were again stimulated with *M. brumae,* BCG and *M. gastri* but, in this case, macrophages were obtained from mouse bone marrow. The induction of different cytokine and chemokine production was evaluated in the supernatant of cultures (Figure 11). In these cultures, there was an increased production of IL-12 and IL-6 cytokines and RANTES and IP-10 chemokines in the supernatants of cells stimulated with *M. brumae* in comparison with the cells stimulated with BCG. In all cases, the production of cytokines induced by *M. brumae* and BCG was higher than that induced by *M. gastri,* except for the production of IP-10, where BCG induces a lower production as compared to the rest of mycobacteria (Figure 11).

The cytotoxic activity of macrophages stimulated with different mycobacteria was also investigated in murine tumor cells. To this effect, murine bladder cancer cell line MB49 was used. It was observed that like in human tumor cells, BCG and *M. brumae* inhibited tumor cell proliferation and induced the production of cytokines (Figure 12).

Finally, the capacity of inducing cytotoxic activity against tumor cells was examined. Like in human cells, the experiments demonstrated that both *M. brumae* and BCG activated the cells of the immune system by inducing cytotoxic activity. In addition, it was observed that the production of soluble factors in the supernatant with cytotoxic activity was also induced (Figure 13).

### Example 17. Pathogenicity

No infections caused by *M. brumae* have been reported either in humans or animals. In the present invention, it was observed that *M. brumae* did not survive in macrophages infected *in vitro.* In contrast, BCG remained in the macrophage over time (Figure 14). Moreover, the viability of mycobacteria within tumor cells was examined. Thus, it was observed that *M. brumae,* together with *M. vaccae* (smooth variant) fail to be viable within T24 tumor cell line after 24-hour incubation (Figure 15).

### Example 18. Comparative assays using mycobacteria other than M. brumae.

The inhibition of proliferation of tumor cell lines, T24 (grade 3), RT112 and 5637 (grade 2) and SW780 (grade 1) was investigated after infection with different mycobacteria. Infection was induced by live mycobacteria at a MOI of 10:1 for 72 hours. Figure 16 shows the results expressed as survival rate in relation to control (uninfected) cells. Each column represents the mean ± standard deviation of culture triplicates from at least three independent experiments.

The following mycobacterium species, as disclosed by Yuksel et al., were used in this assay: *Mycobacterium aichiense* (CR-103), *Mycobacterium aurum* (ATCC 23366), *Mycobacterium brumae* (ATCC 51384), *Mycobacterium chitae* (CIP 141160002), *Mycobacterium chubuense* (ATCC 27278), *Mycobacterium gadium* (ATCC 27726) and *Mycobacterium obuense* (ATCC 27023) obtained from the Collection of Microbial Strains of our laboratory (Laboratory of Mycobacteriology, Autonomous University of Barcelona (UAB), Barcelona, Spain). BCG, *M. brumae, M. chitae* and *M. gadium* were grown in a solid culture medium, i.e., Middlebrook 7H10 agar (Difco) supplemented with 10% OADC at 37°C (excepting *M. gadium* which grew at 30°C). *M. aichiense, M. aurum*, *M. chubuense* and *M. obuense* grew in TSA at 30°C (excepting *M. aichiense* which grew at 37°C). However, the possible use of the above mycobacteria for therapy of bladder cancer is not specifically described by Yuksel et al., as discussed in chapter "State of the Art" of the present invention.

In all the experiments performed, a higher antitumor effect of *M. brumae* versus the rest of mycobacteria under study was observed (except for BCG in T24 cell line). In T24 (grade 1) cell line, *M. brumae* inhibited 34% the proliferation of tumor cells, while BCG induced up to 40% inhibition. Inhibition in the rest of mycobacteria ranged from 4% to 10%, except for *M. chitae* that induced 20% inhibition.

In grade 2 tumor cell lines, the inhibition patterns of mycobacteria are different, but in both cases *M. brumae* induced the highest inhibition rate of cell proliferation, values being even higher than those induced by BCG. In the case of RT112 cell line, *M. brumae* inhibited 20% tumor proliferation, while the rest of mycobacteria under study, including BCG, showed between 1% and 8% inhibition. In 5637 tumor cell line, *M. brumae* inhibited 51% tumor cell proliferation. BCG and *M. chitae* inhibited 36% and 40% cell proliferation, respectively, while the rest of mycobacteria showed lower inhibition values of cell proliferation.

Finally, in SW780 grade 1 cell line, the inhibition of *M. brumae* was between 15 and 20% higher than the inhibition of cell proliferation induced by the rest of mycobacteria (including BCG), reaching a value of 36%.

### Example 19. In vivo experiments. Murine bladder cancer model.

C57BL/6 6-8 week-old female mice were used (Harlan Laboratories, Barcelona, Spain). Mice were kept in quarantine in the animal housing facilities of the Autonomous University of Barcelona for one week.

Animals were housed in groups of four in cages with food and water *ad libitum.* The experimental model of mouse orthotopic bladder cancer, previously described in the literature (Günther JH,1999; Zaharoff DA, 2009) was applied. Animals were cannulated with 24G urethal catheters (Becton Dickinson, Temse, Belgium). Firstly, the animals were intravesically administered with a 0.1 ml solution of poly-L-lysine (molecular weight 70.000-150.000) (Sigma-Aldrich, Madrid, Spain), which remained in the bladder for 10 minutes. Using the same procedure, 10⁵ cells of murine bladder cancer cell line, MB49, in 0.1 ml of fetal bovine serum-free and antibiotic-free DMEM supplemented with glutamine and glucose (PAA Laboratories GmbH, Austria) were subsequently administered. The cell suspension was maintained within the bladder for 60 minutes.

After inducing the tumor, the animals were distributed in three groups depending on the intravesical treatment that they would be later given. According to the scheme in Figure 17, the animals were treated with PBS, 4x10⁶ CFU of *M. bovis* BCG or *M. brumae* in 0.1 ml PBS, on days 1, 8, 15 and 22 after tumor induction. Treatments were performed by intravesical instillations for 60 minutes as indicated above.

All treatments were performed with anesthetized animals (isofluoran 2% O₂). Procedures were in compliance with the Human and Animal Experimentation Ethics Committee, Autonomous University of Barcelona.

Survival of animals submitted to different treatments was compared by using the Kaplan-Meier curves. For calculation of significant differences, the Log-Rank Test was used. A value of p<0.05 was considered significant.

### Example 20. Intravesical treatment with M. brumae or BCG in a murine model

Using the same animal model described in the above example 19, survival of PBS-treated animals (control group) was compared with that of the animals treated by intravesical instillation of study mycobacteria. As shown in Figure 18, animal survival increased significantly when treated with either BCG or *M. brumae* (p <0.05). There were no significant differences between BCG-treated group and *M. brumae-*treated group (p >0.05).

The results obtained in these *in vivo* experiments demonstrate the antitumor capacity of *M. brumae,* thus confirming the results previously obtained in *in vitro* experiments.

### REFERENCES

1. Aguilar JL, Sanchez EE, Carrillo C, Alarcón GS, Silicani A. (1989) Septic arthritis due to Mycobacterium phlei presenting as infantile Reiter's syndrome. J Rheumatol. 16(10): 1377-8.
2. Álvarez Kindelán J., Campos Hernández J.P., López Beltrán A., Requena Tapia M.J. (2007) Clasificación de la OMS 2004 para los tumores vesicales: resumen y comentarios. Actas Urol Esp. 31 (9):978-988.
3. Assersohn L, Sourberbielle B E, O'Brien M E, Archer C D, Mendes R, Bass R, Bromelow K V, Palmer R D, Bouilloux E, Kennard D A, Smith I E. (2002). A randomized pilot study of SRL 172 (Mycobacterium vaccae) in patients with small cell lung cancer (SCLC) treated with chemotherapy. Clin. Oncol. 14:23.
4. Babjuk M, Oosterlinck W, Sylvester R, Kaasinen E, Böhle A, Palou-Redorta. (2008). Guia clinica del carcinoma urotelial no músculo invasive de la Asociación Europea de Urologia. Actas urológicas Españolas. 33:361-371.
5. Babjuk M, Oosterlinck W, Sylvester R, Kaasinen E, Böhle A, Palou-Redorta J, Rouprêt M; European Association of Urology (EAU). (2011) EAU guidelines on non-muscle-invasive urothelial carcinoma of the bladder, the 2011 update. Eur Urol. 59 (6): 997-1008.
6. Bevers RF, et al. (2004) Role of utotherial cells in BCG immunotherapy for superficial bladder cancer. Br J Cancer. 91: 607-12.
7. Böyum A. Separation of leukocytes from blood and bone marrow. Introduction. Scand J Clin Lab Invest Suppl (1968) 97:7.
8. Brandau S, Suttmann H. (2007). Thirty years of BCG immunotherapy for non-muscle invasive bladder cancer: a success story with room for improvement. Biomed. Pharmacother. Rev. 61:299-305.
9. Cage GD, Saubolle MA. (1997) Infrequently isolated and unusual mycobacterial species as agents of human disease. Reviews in Medical Microbiology. 8 (3): 125-135.
10. Dobos KM, Spotts EA, Quinn FD, King CH. Necrosis of lung epithelial cells during infection with Mycobacterium tuberculosis is preceded by cell permeation. Infect Immun (2000) 68(11): 6300-10.
11. Fox CB, Baldwin SL, Duthie MS, Reed SG, Vedvick TS. (2011) Immunomodulatory and physical effects of oil composition in vaccine adjuvant emulsions. Vaccine. 29 (51): 9563-9572.
12. Garcia MM, Brooks BW, Stewart RB, Dion W, Trudel JRR, Ouwerkerk T. Evaluation of gamma radiation levels for reducing pathogenic bacteria and fungi in animal sewage and laboratory effluents. Can J Vat Res (1987) 51: 285-9.
13. Gontero P, Bohle A, Malmstrom P-U, O'Donnell M A, Oderda M, Sylvester R, Witjes F. (2010). The role of Bacillus Calmette-Guérin in the treatment of non-muscle-invasive bladder cancer. Eur. Urol. 57:410-429.
14. Gonzalez OY, Musher DM, Brar I, Furgeson S, Boktour MR, Septimus EJ, Hamill RJ, Graviss EA. (2003) Spectrum of bacille Calmette-Guérin (BCG) infection after intravesical BCG immunotherapy. Clin Infect Dis. 36 (2): 140-8.
15. Gulle H, Fray LM, Gormley EP, Murray A, Moriarty KM. Responses of bovine T cells to fractionated lysate and culture filtrate proteins of Mycobacterium bovis BCG. Vet Immunol Immunopath (1995) 48: 183-90.
16. Günther JH, Jurczok A, Wulf T, Brandau S, Deinert I, Jocham D, and Böhle A. Optimizing Syngeneic Orthotopic Murine Bladder Cancer (MB49). Cancer Res. 1999. 59: 2834-37.
17. Hachem R, Raad I, Rolston KV, Whimbey E, Katz R, Tarrand J, Libshitz H. (1996) Cutaneous and pulmonary infections caused by Mycobacterium vaccae. Clin Infect Dis. 23 (1): 173-175.
18. Houdra D, Baban B, Dunsmuir W, Kirby R, Dalgleish AG. (1998). Immunotherapy of advanced prostate cancer: a phase I/II trial using Mycobacterium vaccae (SRL 172). BR. J. Urol. 13:182.
19. Jin TH, Nguyen L, Qu T, Tsao E. (2011) Improved formulation and lyophilization cycle for BCG vaccine. Vaccine. 29 (29-30): 4848-4852.
20. Khatter S, Singh UB, Arora J, Rana T, Seth P. (2008) Mycobacterial infections in human immuno-deficiency virus seropositive patients: role of non-tuberculous mycobacteria. Indian J Tuberc. 55(1):28-33.
21. Kresowik T P, Griffith T S. (2009). Bacillus Calmette-Guerin immunotherapy for urothelial carcinoma of the bladder. Immunotherapy. 1(2):281-288.
22. Lamm D L. (2000). Efficacy and safety of Bacille Calmette-Guérin immunotherapy in superficial bladder cancer. Clin. Infect. Dis.31:S86-S90.
23. Luquin M., Ausina V., Vincent-Levy-Frebault V., Laneelle M.A., Belda F., Garcia-Barcello M., Prats G., and Daffe M. (1993) Mycobacterium brumae sp. nov., a rapidly growing, nonphotochromogenic mycobacterium. Int. J. Syst. Bacteriol. 43:405-413.
24. Mendes R, O'Brien M E R, Mitra A, Norton A, Gregor R K, Padhani A R, Bromelow K V, Winkley A R, Ashley S, Smith I E, Souberbielle B E. (2002). Clinical and immunological assessment of Mycobacterium vaccae (SRL 172) with chemotherapy in patients with malignant mesothelioma. BR. J. Immunol. 86:336.
25. Mosmann T. Rapid colorimetric assay for cellular growth and survival: Application to proliferation and cytotoxicity assays. J Immunol Methods (1983) 65: 55-63.
26. Nadasy KA, et al. (2008) Four cases of disseminated Mycobacterium bovis infection following intravesical BCG instillation for treatment of bladder carcinoma. South Med. J. 101: 91-95.
27. Ploeg M, et al. (2009) The present and future burden of urinary bladder cancer in the world. World J Urol. 27(3): 289-93.
28. Paul E, Devarajan P. (1998) Mycobacterium phlei peritonitis: a rare complication of chronic peritoneal dialysis. Pediatric nephrology. 12 (1): 67-68.
29. Racoosin EL, Swanson JA. Macropage colony-stimulating factor (rM-CSF) stimulates pinocytosis in bone marrow-derived macrophages. J Exp Med (1989) 170: 1635-48.
30. Rothfuchs AG, Gigliotti D, Palmblad K, Andersson U, Wigzell H, Rottenberg ME. IFN-alpha betadependent, IFN-gamma secretion by bone marrow-derived macrophages controls an intracellular bacterial infection. J Immunol (2001) 167: 6453-61.
31. Saban MR (2008) Molecular networks discriminating mouse bladder responses to intravesical bacillus Calmette-Guerin (BCG), LPS, and TNF-a. BMC Immunol. 9: 4.
32. Shin DM, Yang CS, Yuk JM, Kim KH, Shin SJ, Takahara K, Lee SJ, Jo EK. Mycobacterium abscessus activates the macrophage innate immune response via a physical and functional interaction between TLR2 and dectin-1. Cell Microbiol (2008) 10(8): 1608-21.
33. Shin G A, Lee J K, Freeman R, Cangelosi G A. (2008). Inactivation of Mycobacterium avium complex by UV irradiation. App. Environ. Microbiol. 74:7064-7069.
34. Shintani Y., et al. (2007) Intravesical instillation therapy with bacillus Calmette-Guérin for superficial bladder cancer: Study of the mechanism of BCG immunotherapy. Int J Urol. 14: 140-6.
35. Stokes RW, Norris-Jones R, Brooks DE, Beveridge TJ, Doxsee D, Thorson LM. The glycan-rich outer layer of the cell wall of Mycobacterium tuberculosis acts as an antiphagocytic capsule limiting the association of the bacterium with macrophages. Infect Immun (2004) 72(10): 5676-86.
36. Sobin, LH, Gospodanowicz MK, Wittekind C, editors. TMM classification of malignant tumors (UICC International Union Against Cancer), ed. 7, New York, NY: Wiley-Blackwell; 2009.
37. Suttmann H, Jacobsen M, Reiss K, Jocham D, Bohle A, Brandau S. (2004) Mechanisms of bacillus Calmette-Guerin mediated natural killer cell activation. J Urol. 172: 1490-5.
38. van Rhijn BW, et al. (2009) Recurrence and progression of disease in non-muscle-invasive bladder cancer: from epidemiology to treatment strategy. Eur Urol. 56(3): 430-42.
39. Yuksel et al., (2011) Mycobacterial strains that stimulate the immune system most efficiently as candidates for the treatment of bladder cancer. J Mol Microbiol 2011; 20:24-28.
40. Zaharoff DA, Hoffman BS, Hooper HB, Benjamin CJ Jr, Khurana KK, Hance KW, Rogers CJ, Pinto PA, Schlom J, Greiner JW. Intravesical immunotherapy of superficial bladder cancer with chitosan/interleukin-12. Cancer Res. 2009. 69(15): 6192-9.
41. Zhang Y, Khoo HE, Esuvaranathan K. (1997) Effects of bacillus Calmette-Guerin and interferon a2b on human bladder cancer in vitro. Int. J. Cancer. 71: 851-857.

## Claims

1. Use of *Mycobacterium brumae* for the preparation of a medicament.

2. Use of *Mycobacterium brumae,* according to claim 1, for the preparation of a medicament intended for the treatment of bladder cancer.

3. Use of *Mycobacterium brumae,* according to claim 2, for the preparation of a medicament intended for the treatment of superficial non-invasive bladder cancer.

4. Use of *Mycobacterium brumae,* according to any of claims 1 to 3, for the preparation of a medicament intended for the treatment of grade 1 and grade 2 bladder cancer.

5. Use of *Mycobacterium brumae,* according to any of the above claims, in combination with at least one cytostatic compound.

6. Use of *Mycobacterium brumae,* according to claim 5, wherein the cytostatic compound is mitomycin C.

7. A pharmaceutical composition comprising *Mycobacterium brumae* and pharmaceutically acceptable excipients.

8. A pharmaceutical composition, according to claim 7, comprising *Mycobacterium brumae* in combination with at least one cytostatic compound and pharmaceutically acceptable excipients.

9. A pharmaceutical composition, according to claim 8, wherein the cytostatic compound is mitomycin C.

10. A pharmaceutical composition, according to claim 7, **characterized in that** the pharmaceutically acceptable excipients are selected from the group comprising water, saline, saccharose, mannose, trehalose, sodium glutamate, glycerol and non-ionic and ionic polymers, or combinations thereof.

11. A pharmaceutical composition, according to claims 7 to 10, **characterized in that** said composition is administered by the intranasal, intradermal, oral, vaginal route or through the urinary tract.
